# FASCICULE DE BREVET EUROPEEN

(11) **EP 2 777 709 B1**
(45) Date de publication et mention de la délivrance du brevet: **13.01.2016**
(21) Numéro de dépôt: 14159325.1
(22) Date de dépôt: 13.03.2014
(51) Int. Cl.: A61K 8/97, A61K 36/88, A61Q 19/00

(54) **Utilisation d'un extrait lipophile de jacinthe d'eau pour l'hydratation de la peau**
Verwendung eines lipophilen Wasserhyazinthen-Extrakts zur Versorgung der Haut mit Feuchtigkeit
Use of a lipophilic extract of water hyacinth for moisturising the skin

(30) Priorité: 15.03.2013 FR 1352325
(43) Date de publication de la demande: 17.09.2014
(73) Titulaire: SOCIETE DE RECHERCHE COSMETIQUE SARL, 2314 Luxembourg (LU)
(72) Inventeur: Leconte, Nadine, 92600 ASNIERES SUR SEINE (FR); Rossignol-Castera, Anne, 34160 RESTINCLIERES (FR)
(74) Mandataire: Goulard, Sophie

(56) Documents cités:
- WO-A1-01/87261
- WO-A1-01/87319
- WO-A1-2010/112760
- DE-A1-102009 049 680
- JP-A- 2004 123 660
- Alban Muller: "Ingrédients cosmétiques écoresponsables", , 2012, pages 1-29, XP002699542, Extrait de l'Internet: URL:http://www.albanmuller.com/cosmetique/ Ingredients-Cosmetiques-Naturels-AMI.pdf [extrait le 2013-06-26]

## Description

La présente invention concerne une nouvelle composition cosmétique destinée à maintenir ou rétablir l'hydratation de la peau, et plus particulièrement une nouvelle utilisation d'un extrait lipophile de jacinthe d'eau, ou Eichhornia crassipes, dans une composition cosmétique à effet hydratant.

L'eau est un constituant majoritaire de l'organisme humain et représente généralement 60 à 65% du poids corporel d'un individu adulte. Une partie de cette eau, soit 6 à 8 l environ, est présente dans la peau, principalement dans le derme où elle où elle forme un gel semi-fluide avec des glycosaminoglycanes (GAGs) et des glycoprotéines de structure. On considère que l'épiderme contient environ 120 ml d'eau tandis que la couche cornée n'en contient que 20 ml chez un adulte. La peau comprend en effet plusieurs couches intégrées, allant de la couche superficielle, la couche cornée disposée sur l'épiderme, jusqu'aux couches plus profondes, le derme et l'hypoderme, chacune possédant des propriétés spécifiques permettant à l'ensemble de réagir et de s'adapter aux conditions de son environnement.

La couche cornée, ou stratum corneum, est essentiellement constituée de cornéocytes, et, en raison de son pH, de sa faible teneur en eau, de la présence de peptides antibactériens ayant une action bactéricide et d'une flore bactérienne commensale, joue le rôle de première défense antimicrobienne. L'épiderme, principalement composé de kératinocytes, de mélanocytes et des cellules de Langerhans, joue un rôle fondamental pour assurer la protection et le maintien d'une bonne trophicité.

Le derme se compose principalement de collagène, d'élastine et de protéoglycanes, molécules synthétisées par les fibroblastes dermiques. Les fibres de collagène assurent la résistance mécanique et la texture de la peau, l'élastine est responsable de l'élasticité, et les protéoglycanes, combinant protéine et glycosaminoglycane, jouent un rôle majeur de structure et d'hydratation de la peau. Les glycosaminoglycanes, tels que l'acide hyaluronique, sont des molécules très hygroscopiques qui sont capables de retenir des quantités importantes d'eau. D'autres cellules comme les macrophages et les leucocytes sont également présentes dans la couche du derme. L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

La couche cornée peut laisser passer d'infimes quantités d'eau, et ces pertes peuvent être amplifiées dans certaines conditions comme par exemple une forte exposition aux rayons ultraviolets. De plus, une dérégulation des divers composants de la peau ou certaines pathologies comme l'atopie et la xérose, peuvent entraîner des troubles de l'hydratation. Le vieillissement de la peau s'accompagne aussi d'une plus forte sécheresse généralement liée à une moindre sécrétion de sébum ou à des diminutions des flux hydriques à travers l'épiderme. La sécheresse de la peau entraîne généralement des démangeaisons et des sensations désagréables et inconfortables. C'est pourquoi de nombreuses compositions cosmétiques ont été proposées pour prévenir ou limiter la déshydratation de la peau.

On connaît ainsi l'utilisation de divers polyols, comme le glycérol ou le butylène glycol, des sucres tels que le maltitol, des mucopolysaccharides, ou l'acide hyaluronique. On peut aussi améliorer l'effet d'hydratation en associant un agent hydratant tel que le glycérol à un polysiloxane réticulé comme décrit dans la demande WO 0215872. On a aussi proposé d'utiliser certains extraits de plantes, par exemple un hydrolysat peptidique de pois comme dans le brevet FR 2.958.847, ou un extrait de pulpe de grenade comme dans le brevet FR 2.874.502.

La jacinthe d'eau (Eichhornia crassipes) est une plante aquatique de la famille des Pontederiaceae, originaire de la zone amazonienne, qui s'est développée dans diverses régions tropicales et subtropicales où sa croissance rapide, parfois de 2 à 5 m par jour, en fait une plante invasive dont les tiges forment des tapis flottants denses et dont les feuilles se tiennent sur les tiges, au-dessus de la surface de l'eau. Les fleurs, rassemblées en épis de 8 à 15 fleurs au sommet des tiges, comportent chacune six pétales de couleur bleu violacé à rose, le pétale du sommet étant jaune. Le fruit de la jacinthe d'eau forme une capsule pouvant contenir plus de 300 graines.

La rapidité de sa croissance fait de la jacinthe d'eau une plante aquatique invasive provoquant des effets néfastes sur les écosystèmes en limitant la lumière nécessaire à la vie aquatique. Elle a parfois été utilisée pour l'alimentation du bétail, pour la fabrication de meubles artisanaux en Asie et en Afrique, ou pour le traitement des eaux usées en raison de la capacité de ses racines à absorber les métaux lourds.

Les rendements atteignent 200 tonnes de matière sèche par hectare et par an, les protéines représentant 20 à 25 % de cette matière sèche. Ces rendements sont exceptionnellement élevés. Après fauchage, le limbe représente 30 % de la matière fraiche et le reste de la plante 70 %. Le pressage conduit à l'obtention d'un jus contenant 1,5 % de matière sèche (60 % de matière azotée et 35 % de minéraux) tandis que le tourteau contient 35 % de matière sèche (20 % de matières azotées totales, 10 % de minéraux, des fibres des lipides dont beaucoup de cires).

La jacinthe d'eau peut accumuler de nombreux composants polluants, et elle agit alors sur son environnement comme agent détoxifiant. Mais cette accumulation de polluants génère des dérivés actifs de l'oxygène dans les cellules de la jacinthe d'eau. L'antioxydant majeur des cellules, le glutathion sous forme réduite, va alors protéger les cellules des radicaux libres. La jacinthe d'eau possède donc une activité anti-oxydante et la quantité de glutathion présente dans ses feuilles a été estimée à 40 nmoles par gramme de plante sèche.

Divers travaux ont été réalisés pour tenter de valoriser la jacinthe d'eau et par exemple, la demande de brevet US 2005/214389 décrit un procédé d'extraction de β-carotène à partir d'Eichhornia crassipes dans un solvant organique après séchage et broyage de la plante. Une activité hypolipidémiante d'un extrait de Eichhornia crassipes a été décrite dans le brevet CN 101337009. La demande de brevet WO 0187319 décrit l'utilisation d'extraits cellulaires de jacinthe d'eau dans des compositions cosmétiques comme agent anti-pollution ou de dépollution pour protéger la peau des effets nocifs des métaux lourds, tels que cadmium, nickel et plomb présents dans l'environnement. Cependant, la possibilité d'utiliser des extraits lipophiles de Eichhornia crassipes, ou jacinthe d'eau, pour favoriser l'hydratation de la peau n'a jamais été suggérée.

Bien que de nombreuses compositions cosmétiques aient été proposées, il existe toujours un besoin de pouvoir disposer de nouvelles compositions topiques alternatives permettant de maintenir ou de rétablir l'hydratation de la peau, et notamment des compositions topiques à base d'extraits végétaux appropriés provenant plus particulièrement de plantes connues pour leurs propriétés favorables à une telle activité.

Les études effectuées par la demanderesse ont montré qu'il est possible de maintenir et rétablir l'hydratation de la peau en utilisant des compositions topiques à base d'extraits lipophiles de Eichhornia crassipes, ou jacinthe d'eau. En effet, les études ont montré qu'un extrait lipophile de Eichhornia crassipes possède une action efficace comme agent hydratant dans des compositions cosmétiques topiques.

La présente invention a donc pour objet une nouvelle utilisation cosmétique d'au moins un extrait lipophile de Eichhornia crassipes à titre d'agent hydratant.

Un autre objet de l'invention est une composition huileuse renfermant au moins un extrait lipophile de Eichhornia crassipes dans un support huileux comprenant au moins une huile végétale naturelle, raffinée ou non choisie parmi l'huile de tournesol, l'huile d'olive, l'huile de pépins de raisin et l'huile de jojoba.

La présente invention a encore pour objet un procédé cosmétique non thérapeutique pour procurer une action topique hydratante, consistant à appliquer sur les zones de la peau concernées une composition cosmétique contenant une quantité efficace d'une composition huileuse renfermant au moins extrait lipophile de Eichhornia crassipes selon la présente invention.

Les compositions utilisées dans la présente invention se distinguent en ce qu'elles comprennent une composition huileuse renfermant au moins un extrait lipophile de Eichhornia crassipes en une quantité efficace pour procurer un effet d'hydratation de la peau, en particulier du derme et de l'épiderme, ainsi que des supports et excipients acceptables en cosmétologie.

Ainsi, les compositions topiques de l'invention à base d'extraits lipophile de Eichhornia crassipes peuvent être utilisées avantageusement en cosmétologie pour le traitement et la prévention des sécheresses cutanées se traduisant par des sensations désagréables et inconfortables.

Les études effectuées par la demanderesse ont montré que l'on peut utiliser toutes les parties de la plante, aussi bien les racines que les parties aériennes, notamment les écorces, les tiges, les feuilles, les fleurs, les fruits et les graines, mais on utilise de préférence les parties aériennes. L'espèce *Eichhornia crassipes* est facilement disponible et la conservation des plantes ne soulève généralement pas de difficulté technique.

Selon chaque plante ou organe de plante, un extrait particulier peut être réalisé afin d'optimiser le rendement d'extraction des molécules ou familles de molécules recherchées.

Dans la présente demande, le terme "extrait" doit s'entendre comme "extrait de cellules des plantes" du genre Eichhornia, et plus particulièrement Eichhornia crassipes ou jacinthe d'eau.

L'extrait lipophile de Eichhornia crassipes peut être obtenu selon le procédé d'extraction décrit dans la demande de brevet WO 2010112760 consistant, dans une première étape, à mélanger et imprégner la plante, préalablement séchée et broyée, dans un corps gras naturel à une température supérieure à son point de fusion, puis à chauffer le mélange à haute température pendant une durée très courte, et à former une microdispersion à une température supérieure à la température de fusion du corps gras. Toutes ces étapes de font de préférence à l'abri de l'air, plus particulièrement à l'abri de l'oxygène pour éviter toute réaction d'oxydation de l'extrait. On peut par exemple travailler sous atmosphère d'azote, dans un réacteur clos avec extraction continue de l'oxygène par un flux d'azote, ou encore sous vide.

Le corps gras utilisé dans ce procédé peut être une huile végétale naturelle, raffinée ou non, comprenant au moins 0,1% en poids d'eau, par exemple une huile de tournesol, d'olive, de pépins de raisin, ou de jojoba. Suivant l'invention, on utilise de préférence une huile de jojoba, qui est une cire naturelle liquide à température ambiante, constituée à près de 98% d'esters aliphatiques insaturés d'acides gras et d'alcools gras mono-insaturés, majoritairement en C₂₀ et C₂₂. Sa composition est proche de celle du sébum de la peau, ce qui lui confère des propriétés nutritives et émollientes remarquables et une parfaite tolérance cutanée.

L'utilisation d'une huile végétale comme solvant de l'extrait de Eichhornia crassipes présente l'avantage d'être compatible avec les normes des produits certifiés Ecocert s'appliquant aux produits cosmétiques à la fois écologiques et biologiques.

Ainsi, selon l'invention, l'extrait lipophile de Eichhornia crassipes se présente de préférence sous la forme d'une composition huileuse renfermant ledit extrait lipophile dans un support huileux comprenant au moins une huile végétale naturelle, raffinée ou non choisie parmi l'huile de tournesol, l'huile d'olive, l'huile de pépins de raisin et l'huile de jojoba. Selon une forme de réalisation particulièrement préférée de l'invention, l'huile végétale est l'huile de jojoba.

La première étape du procédé ci-dessus, consistant à imprégner la plante, de préférence broyée, se déroule à température ambiante, ou à chaud, à une température supérieure à celle de fusion du corps gras utilisé. On travaille de préférence à température ambiante, de l'ordre de 25°C, dans une huile végétale liquide à cette température.

La deuxième étape comporte un chauffage à une température élevée, par exemple de l'ordre de 140 à 170°C, pendant une courte durée, généralement inférieure à 5 minutes. Le temps de montée en température doit aussi être très court. La technique du chauffage par micro-ondes est bien adaptée à ces conditions. La technique des micro-ondes facilite aussi la troisième étape de microdispersion des particules dans le corps gras, avec rupture des membranes cellulaires. Cette troisième étape peut aussi comprendre un traitement par cavitation ultrasonore, de préférence dans un réacteur fermé équipé d'un générateur d'ultrasons à basse fréquence. Cette troisième étape peut éventuellement être réalisée simultanément avec les deux autres, l'ensemble des étapes, ou chacune d'elles, pouvant être réalisé plusieurs fois si nécessaire.

Ce procédé est particulièrement efficace sur une matière première telle que la jacinthe d'eau contenant entre 80 et 90% d'eau avant broyage et séchage. Après séchage, avant l'étape d'imprégnation dans l'huile végétale, la teneur en eau est ramenée en dessous de 10%.

On obtient ainsi un extrait lipophile de couleur vert foncé, soluble dans les huiles et phases grasses, comprenant principalement les substances suivantes :

| | |
|---|---|
| quercétine | min. 50 mg/kg |
| acide chlorogénique | min. 150 mg/kg |
| oleuropéine | min. 250 mg/kg |

Les analyses ont aussi mis en évidence la présence de glutathion, de cystéine et de lysine. Le glutathion est un tripeptide résultant de la condensation d'acide glutamique, de cystéine et de glycine, qui renforce les défenses immunitaires en inhibant la toxicité des radicaux libres et contribue à éliminer les métaux lourds éventuellement présents par formation de liaisons soufre - métal et élimination par excrétion.

De plus, la technique utilisée permet d'obtenir un extrait huileux bien plus concentré en principes actifs qu'un simple macérât huileux. Ainsi, suivant l'invention on associe les avantages d'une huile végétale et de molécules hydrophiles (flavonoïdes, composés phénoliques). De plus, le support huileux évite de devoir ajouter un conservateur à la composition.

Les extraits de jacinthe d'eau utilisés dans les compositions de la présente invention peuvent être obtenus à partir de chacune des parties accessibles de la plante, telles que feuilles, tiges, racines, fleurs et graines, ou à partir de la plante entière. Suivant l'invention on utilise de préférence les parties aériennes, plus particulièrement les feuilles, les fleurs, les très petites tiges et les flotteurs.

L'extrait lipophile de Eichhornia crassipes peut ensuite être lyophilisé pour faciliter sa conservation.

Les extraits lipophiles de Eichhornia crassipes inclus dans les compositions suivant la présente invention ont montré dans les tests in vitro, décrits plus en détail ci-après, une absence de cytotoxicité et un net pouvoir hydratant. Les tests d'utilisation ont mis en évidence un excellent effet hydratant sans effet secondaire néfaste et avec une excellente tolérance cutanée.

Les extraits lipophiles de Eichhornia crassipes utilisés dans les compositions de l'invention sont généralement en une concentration de l'ordre de 0,05 à 15,0%, de préférence entre 0,2 et 10%, par rapport au poids total de la composition.

Le choix de la concentration en extrait lipophile dans la composition de l'invention peut être fait en fonction de l'utilisation envisagée et du mode d'administration. Pour un traitement réhydratant de confort, on utilise de préférence des doses plus faibles, sous forme de lait ou de crème dosée à environ 1 à 5%, tandis qu'un traitement ponctuel destiné à traiter une sécheresse cutanée sévère peut nécessiter des doses plus élevées, par exemple un sérum dosé entre 5 et 15%, où, de préférence, les doses d'extraits lipophiles de Eichhornia crassipes sont plus élevées.

Ainsi, ces compositions topiques peuvent être utilisées avantageusement en cosmétologie pour le traitement ou la prévention des sensations de démangeaison résultant de sécheresse cutanée. Ces compositions peuvent être utilisées quotidiennement, une à plusieurs fois par jour, sans limitation de durée de traitement.

Les compositions cosmétiques de la présente invention possèdent une excellente tolérance cutanée, notamment en raison du film lipidique résultant du support huileux (huile de jojoba), elles ne présentent aucune phototoxicité et leur application sur la peau, pour des périodes de temps prolongées, n'implique aucun effet systémique.

Les compositions selon l'invention peuvent contenir, outre un extrait lipophile de Eichhornia crassipes, des actifs secondaires complétant avantageusement son activité, et compatibles, c'est-à-dire non susceptibles de réagir les uns sur les autres ou de masquer ou limiter leurs effets respectifs. Les principes actifs complémentaires sont choisis pour leur action, par exemple pour la protection solaire, l'effet anti-rides, l'activité anti-radicalaire et anti-oxydante, l'activité anti-irritante, la nutrition cellulaire, pour le renforcement de l'effet repulpant et de la fermeté de la peau, ainsi que d'autres principes actifs pouvant avoir une action complémentaire sur l'hydratation de la peau.

Plus particulièrement, les actifs secondaires peuvent être choisis par exemple parmi un extrait de graines de mimosa et un extrait de graines de souci agissant favorablement sur la stimulation des collagènes néoformés, un extrait de pétales de coquelicot agissant sur la nutrition cellulaire, des extraits de feuilles pommier ou de cellules de cacao pour leur action sur les glycosaminoglycanes, du monométhylsilanol, de la proline, ou encore du beurre de Karité.

Les compositions conformes à la présente invention peuvent être présentées sous les formes galéniques classiquement utilisées pour une application topique, c'est-à-dire sous forme de lotion, gel, émulsion (en particulier crème ou lait), émulsion biphasique huile-dans-eau ou eau-dans-huile, huile corporelle, masque, onguent, pommade, bâtons protecteur pour les lèvres, nanocapsules, liposomes ou encore des patches transdermiques, contenant des excipients et supports usuels compatibles et acceptables sur le plan cosmétologique. Elles sont utilisées de préférence sous forme de crèmes, huiles, laits et sérums.

Ces formes d'administration par voie topique sont préparées par les techniques usuelles, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout.

Les compositions cosmétiques suivant l'invention peuvent aussi prendre la forme de lotion ou solution dans laquelle les extraits sont sous forme encapsulée. Les microsphères suivant l'invention peuvent par exemple être constituées de corps gras, d'agar et d'eau. Les extraits peuvent être incorporés dans des vecteurs de type liposomes, glycosphères, dans des chylomicrons, des macro-, micro-, nano-particules ainsi que les macro-, micro- et nanocapsules et aussi être adsorbés sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50 °C sans qu'il y ait sédimentation des constituants ou séparation des phases.

Les compositions topiques selon l'invention peuvent comprendre des excipients appropriés pour une administration topique externe, en particulier des excipients acceptables sur le plan dermatologique et cosmétologique. Ces excipients appropriés pour la formulation sont bien connus de l'homme du métier et comprennent en particulier des épaississants tels que les gommes naturelles et les polymères de synthèse ; des gélifiants tels que des esters de cellulose et le carbopol, des émollients et des tensioactifs tels que l'octanoate de cétéaryle, le myristate d'isopropyle, l'isononanoate de cétéaryle, la diméthicone, le 3-diisostéarate de polyglycéryle, l'alcool cétylique, le palmitate cétylique ; des agents antibactériens, des parfums ; etc.

D'autres ingrédients utilisables dans les compositions de l'invention sont les agents hydratants complémentaires tels que la glycérine, l'urée, l'acide hyaluronique de haut poids moléculaire, le butylène glycol, le sel de sodium de l'acide pyrrolidone carboxylique (sodium PCA), et également les vitamines antioxydantes telles que la vitamine E, par exemple l'acétate de tocophérol ou le tocotriénol, la vitamine C, les polyphénols naturels.

On peut également ajouter à la composition des agents de protection contre les rayons ultraviolets, et par exemple des filtres solaires UV-A et UV-B hydrophiles ou lipophiles, choisis parmi des dérivés du dibenzoylméthane tels que le 4-isopropyl dibenzoylméthane (Eusolex® 8020) ou le t-butyl-méthoxy dibenzoylméthane (Parsol® 1789), des dérivés de benzophénone tels que la 2-hydroxy-4-méthoxy-benzophénone (Eusolex® 4360) et le diethylamino hydroxy benzoyl hexyl benzoate (Uvinul A+®), des esters d'acide cinnamique tels que le méthoxycinnamate d'octyle (Eusolex® 2292) ou le méthoxycinnamate d'éthyl-2-hexyle (Parsol MCX®), des dérivés salicyliques tels que le salicylate d'éthylhexyle (Neo Heliopan OS), des dérivés de triazine tels que la bis-éthylhexyloxyphénol méthoxyphényl triazine (Tinosorb® S) ou l'éthylhexyltriazone (Uvinul® T150), le 4-méthylbenzylidène camphre (Eusolex® 6300), ou encore un cyano-β,β-diphénylacrylate tel que l'octocrylène (Eusolex® OCR), etc. On peut aussi utiliser des pigments formant écran anti-ultraviolet, comme par exemple le dioxyde de titane, l'oxyde de zinc, ou l'oxyde de zirconium.

L'utilisation cosmétique d'extraits comprend tous les soins du corps et de la peau y compris les produits solaires, protecteurs et bronzants, les produits anti-âge, anti-séborrhéiques, toniques, les produits assurant l'amélioration de l'aspect de la peau y compris le traitement acnéique et les rougeurs cutanées.

Les exemples suivants illustrent l'invention plus en détail sans en limiter la portée. Dans tous les exemples de compositions qui suivent, les parties sont exprimées en poids, sauf indication contraire.

### EXEMPLES

### Exemple 1

Un extrait lipophile de jacinthe d'eau a été préparé comme indiqué ci-après.

On a utilisé des jacinthes d'eau, plante entière, préalablement broyées et séchées à froid. La technique du séchage à froid est préférable car elle permet de préserver les anti-oxydants présents dans la plante. Suivant cette technique, les plantes ont été soumises à un premier broyage, puis à une déshydratation à température ambiante à l'abri de la lumière, de manière à ramener la teneur en eau vers 10%, et broyées à froid, à une température de -80°C, au moyen d'un broyeur à couteaux.

La teneur en eau était de 7,0% après broyage et séchage à froid.

1 kg de jacinthe d'eau ainsi séchée et broyée, sous forme de poudre fine et homogène, a été mélangé à 5 L d'huile vierge de jojoba. Le rapport plante / huile était d'environ 1/8. Le mélange a été maintenu sous flux d'azote pendant 4 heures à température ambiante.

Le mélange a ensuite été chauffé rapidement par micro-ondes (1.000 watts, 2 x 3 min.), sous flux d'azote, la température maximale atteinte étant de 150°C.

Le mélange a ensuite été traité par ultrasons (20 kHz), sous azote et sous agitation, pendant 10 minutes. L'huile contenant l'extrait lipophile de Eichhornia crassipes a été séparée par centrifugation.

### Exemple 2

Cet exemple présente le contrôle de l'absence de cytotoxicité de l'extrait lipophile de Eichhornia crassipes suivant l'invention.

Le composé testé est l'extrait de jacinthe pur, tel qu'obtenu par le procédé de l'Exemple 1. Le test sur la viabilité d'épidermes humains reconstruits utilisé est celui de la réduction au bleu de Formazan (test MTT) après 24 heures d'incubation de cellules de kératinocytes. On a utilisé deux lots, où le lot n°1 (témoin) était un épiderme non traité tandis que le lot n°2 était un épiderme traité par l'extrait pur.

Les résultats sont indiqués dans le tableau 1 ci-après :

**TABLEAU 1**

| **Substance** | **Densité optique DO 540 nm** | **%** |
|---|---|---|
| **Lot n°1 (témoin)** | 0,398 ± 0,05 | - |
| **Lot n°2 (extrait pur)** | 0,437 ± 0,05 | ns |

Aucune cytotoxicité n'a été observée par rapport au témoin.

### Exemple 3

### Activité hydratante d'un extrait lipophile de Eichhornia crassipes

Le but de cette étude était de déterminer l'effet hydratant d'un extrait lipophile suivant l'invention.

L'effet hydratant a été évalué par la mesure de la diffusion de la caféine à partir d'un épiderme humain reconstruit. La diffusion de la caféine est inversement proportionnelle à la fonction barrière. Un composant renforçant cette barrière cutanée est donc un composant qui diminue la diffusion de la caféine.

La diffusion de l'extrait lipophile de jacinthe d'eau a été testée à partir d'épidermes pré-traités en caféine, afin de vérifier l'impact sur la barrière cutanée.

Les résultats obtenus sont présentés dans le tableau 2 ci-après :

**TABLEAU 2**

| **Traitement** | **Composé testé** | **cpm (moyenne)** | **Fonction Barrière** |
|---|---|---|---|
| | Témoin | 4853 ± 168 | 100 |
| 30 min | Vitamine C | 3487 ± 196 | 140 |
| | Extrait Jacinthe | 4387 ± 237 | 111 |
| | Témoin | 15025 ± 696 | 100 |
| 1 heure | Vitamine C | 10719 ± 785 | 141 |
| | Extrait Jacinthe | 13621 ± 1066 | 111 |
| | Témoin | 32893 ± 2123 | 100 |
| 2 heures | Vitamine C | 24441 ± 435 | 134 |
| | Extrait Jacinthe | 25831 ± 1622 | 127 |
| | Témoin | 44526 ± 2207 | 100 |
| 3 heures | Vitamine C | 35877 ± 637 | 124 |
| | Extrait Jacinthe | 36121 ± 1832 | 123 |
| | Témoin | 62318 ± 2038 | 100 |
| 4 heures | Vitamine C | 50255 ± 448 | 124 |
| | Extrait Jacinthe | 47924 ± 1669 | 130 |
| | Témoin | 73639 ± 4149 | 100 |
| 5 heures | Vitamine C | 59780 ± 1167 | 123 |
| | Extrait Jacinthe | 55374 ± 795 | 133 |
| | Témoin | 77391 ± 1512 | 100 |
| 6 heures | Vitamine C | 63598 ± 643 | 122 |
| | Extrait Jacinthe | 61501 ± 2375 | 126 |
| | Témoin | 85130 ± 410 | 100 |
| 7 heures | Vitamine C | 70889 ± 1868 | 120 |
| | Extrait Jacinthe | 68166 ± 3136 | 125 |
| | Témoin | 87914 ± 1225 | 100 |
| 8 heures | Vitamine C | 77191 ± 2408 | 114 |
| | Extrait Jacinthe | 72594 ± 2774 | 121 |

Le témoin est un épiderme non traité. La Vitamine C a été utilisée à la dose de 200 µg/ml. L'extrait lipophile de jacinthe d'eau suivant l'invention a été utilisé pur et contenait 500 µM de quercétine. La fonction barrière a été mesurée en % par rapport au témoin.

Le tableau 2 ci-dessus montre que le passage de la caféine à travers les épidermes témoins s'est effectué de façon progressive tout au long de la cinétique, et que le traitement à la vitamine C, testée à 200 µg/ml, a réduit de façon significative cette diffusion, traduisant ainsi une augmentation de la fonction barrière des épidermes. Les effets de la référence (Vitamine C), bien que présents tout au long de la cinétique, étaient plus marqués durant les premiers temps d'analyse (entre 30 min et 4 heures).

L'extrait lipophile de jacinthe d'eau suivant l'invention, tel que préparé suivant l'exemple 1, a présenté un effet stimulant sur la fonction barrière des épidermes reconstruits en réduisant la diffusion de la caféine. Cet effet significatif est observé tout au long de la cinétique à partir du temps 3 heures.

Ces essais démontrent l'effet hydratant de l'extrait lipophile de jacinthe d'eau suivant l'invention, en renforçant la cohésion de la barrière cutanée et donc en maintenant l'eau au sein des tissus, pendant une durée de 8 heures.

### Exemple 4

Par les techniques usuelles, on a préparé une émulsion solaire ayant la composition indiquée ci-après.
- Glycérine 3,0
- EDTA tétrasodique 0,1
- Capryloyl glycine 0,5
- Dibutyl adipate (Cetiol B) 10,0
- C₁₂-C₁₅ alkyl benzoate (Cetiol AB) 10,0
- Coco glucoside 2,0
- Polyacrylate Crosspolymer 6 1,0
- Ethylhexyl triazone 2,0
- Bis-éthylhexylphénol méthoxyphényl triazine 3,0
- Diéthylamino hydroxybenzoyl hexyl benzoate 6,0
- Extrait lipophile de jacinthe d'eau (Eichhornia crassipes) 1,0
- Tocophérol 0,7
- Parfum 1,0
- Eau déminéralisée qsp 100,0

L'extrait lipophile de jacinthe d'eau utilisé dans cet exemple est celui obtenu par le procédé de l'Exemple 1.

Cette émulsion est à utiliser une à deux fois par jour par application sur les zones de la peau à traiter.

### Exemple 5

Par les techniques usuelles, on a préparé une huile solaire ayant la composition indiquée ci-après.
- Dibutyl adipate (Cetiol B) 10,0
- Ethylhexyl triazone 5,0
- Bis-éthylhexylphénol méthoxyphényl triazine 1,0
- Diéthylamino hydroxybenzoyl hexyl benzoate 6,0
- Extrait lipophile de jacinthe d'eau (Eichhornia crassipes) 0,5
- Tocophérol 0,5
- Parfum 1,0
- C₁₂-C₁₅ alkyl benzoate (Cetiol AB) 10,0
- Coco-caprylate/caprate (Dub 810C) qsp 100,0

L'extrait lipophile de jacinthe d'eau utilisé dans cet exemple est celui obtenu par le procédé de l'Exemple 1.

Cette huile est à utiliser une à trois fois par jour par application sur les zones de la peau à protéger.

### Exemple 6

Par les techniques usuelles, on a préparé un sérum ayant la composition indiquée ci-après.
- Glycérine 3,0
- EDTA tétrasodique 0,1
- Capryloyl glycine 0,5
- Acrylate C₁₀-C₃₀ alkyl acrylate Crosspolymer 0,3
- Phényl triméthicone 1,0
- Extrait lipophile de jacinthe d'eau (Eichhornia crassipes) 5,0
- Tocophérol 0,7
- Parfum 0,5
- Eau qsp 100,0

L'extrait lipophile de jacinthe d'eau utilisé dans cet exemple est celui obtenu par le procédé de l'Exemple 1.

Ce sérum est à utiliser plusieurs fois par jour, selon les nécessités, par application sur les zones de la peau à traiter.

## Revendications

1. Utilisation d'un extrait lipophile de Eichhornia crassipes dans une composition cosmétique pour application topique à action hydratante de la peau.

2. Utilisation selon la revendication 1, **caractérisée en ce que** ledit extrait est obtenu par extraction à l'abri de l'air.

3. Utilisation selon la revendication 1, **caractérisée en ce que** l'extrait comprend :
| | |
|---|---|
| quercétine | min. 50 mg/kg |
| acide chlorogénique | min. 150 mg/kg |
| Oleuropéine | min. 250 mg/kg |

4. Utilisation selon l'une quelconque des revendications précédentes, **caractérisée en ce que** ledit extrait se présente sous la forme d'une composition huileuse renfermant ledit extrait lipophile dans un support huileux comprenant au moins une huile végétale naturelle, raffinée ou non choisie parmi l'huile de tournesol, l'huile d'olive, l'huile de pépins de raisin et l'huile de jojoba.

5. Utilisation selon la revendication 4, **caractérisée en ce que** l'huile végétale est l'huile de jojoba.

6. Utilisation selon l'une quelconque des revendications précédentes, **caractérisée en ce que** les extraits sont obtenus à partir de la plante entière.

7. Utilisation selon l'une quelconque des revendications précédentes, **caractérisée en ce que** la composition cosmétique comprend entre 0,05 et 15% en poids d'extrait lipophile de Eichhornia crassipes par rapport au poids total de la composition cosmétique.

8. Utilisation selon la revendication 7, **caractérisée en ce que** la composition cosmétique comprend de 0,2 à 10,0% en poids d'extrait lipophile de Eichhornia crassipes par rapport au poids total de la composition cosmétique.

9. Utilisation selon l'une quelconque des revendications 1 à 6, **caractérisée en ce que** la composition cométique comprend en outre un ou plusieurs actifs secondaires choisis parmi un extrait de graines de mimosa, un extrait de graines de souci, un extrait de pétale de coquelicot, un extrait de feuille de pommier, un extrait de cellules de cacao, du monométhylsilanol, de la proline et du beurre de Karité.

10. Utilisation selon l'une quelconque des revendications précédentes, caractérisée en ce la composition cosmétique est formulée sous forme de lotion, gel, émulsion, émulsion biphasique huile-dans-eau ou eau-dans-huile, huile corporelle, masque, onguent, pommade, bâton protecteur pour les lèvres, nanocapsules, liposomes ou patch transdermique pour application topique.

11. Composition huileuse **caractérisée en ce qu'**elle renferme au moins un extrait lipophile de Eichhornia crassipes dans un support huileux comprenant au moins une huile végétale naturelle, raffinée ou non choisie parmi l'huile de tournesol, l'huile d'olive, l'huile de pépins de raisin et l'huile de jojoba.

12. Procédé cosmétique non thérapeutique pour procurer une action topique hydratante de la peau, consistant à appliquer sur les zones de la peau concernées une composition cosmétique contenant une quantité efficace d'une composition huileuse renfermant au moins un extrait lipophile de Eichhornia crassipes, ladite composition étant telle que définie à la revendication 11.

## Patentansprüche

1. Verwendung eines lipophilen Wasserhyazinthen-Extrakts in einer kosmetischen Zusammensetzung für topische Anwendung mit hydratisierender Wirkung der Haut.

2. Verwendung nach Anspruch 1, **dadurch gekennzeichnet, dass** der Extrakt vor Luft geschützt durch Extraktion hergestellt ist.

3. Verwendung nach Anspruch 1, **dadurch gekennzeichnet, dass** der Extrakt umfasst:
| | |
|---|---|
| Quercetin | mind. 50 mg/kg |
| Chlorogensäure | mind. 150 mg/kg |
| Oleuropein | mind. 250 mg/kg |

4. Verwendung nach einem der vorangehenden Ansprüche, **dadurch gekennzeichnet, dass** der Extrakt in Form einer öligen Zusammensetzung vorliegt, die den lipophile Extrakt in einem öligen Träger einschließt, der mindestens ein natürliches, raffiniertes oder nicht raffiniertes Pflanzenöl, ausgewählt aus dem Sonnenblumenöl, dem Olivenöl, dem Traubenkernöl und dem Jojobaöl, umfasst.

5. Verwendung nach Anspruch 4, **dadurch gekennzeichnet, dass** das Pflanzenöl das Jojobaöl ist.

6. Verwendung nach einem der vorangehenden Ansprüche, **dadurch gekennzeichnet, dass** die Extrakte aus der gesamten Pflanze gewonnen sind.

7. Verwendung nach einem der vorangehenden Ansprüche, **dadurch gekennzeichnet, dass** die kosmetische Zusammensetzung zwischen 0,05 und 15 Gew.-% liophilen Wasserhyazinthen-Extrakt im Verhältnis zum Gesamtgewicht der kosmetischen Zusammensetzung umfasst.

8. Verwendung nach Anspruch 7, **dadurch gekennzeichnet, dass** die kosmetische Zusammensetzung 0,2 bis 10,0 Gew.-% liophilen Wasserhyazinthen-Extrakt im Verhältnis zum Gesamtgewicht der kosmetischen Zusammensetzung umfasst.

9. Verwendung nach einem der Ansprüche 1 bis 6, **dadurch gekennzeichnet, dass** die kosmetische Zusammensetzung ferner einen oder mehrere sekundäre Wirkstoffe umfasst, die aus einem Mimosensamenextrakt, einem Ringelblumensamenextrakt, einem Mohnblumenblütenblätterextrakt, einem Apfelbaumblattextrakt, einem Kakaozellenextrakt, Monomethylsilanol, Prolin und Sheabutter ausgewählt sind.

10. Verwendung nach einem der vorangehenden Ansprüche, **dadurch gekennzeichnet, dass** die kosmetische Zusammensetzung in Form von Lotion, Gel, Emulsion, zweiphasiger Öl-in-Wasser- oder Wasser-in-ÖI-Emulsion, Körperöl, Maske, Salbe, Pomade, Schutzstift für die Lippen, Nanokapseln, Liposomen oder transdermischem Patch für die topische Anwendung formuliert ist.

11. Ölige Zusammensetzung, **dadurch gekennzeichnet, dass** sie mindestens einen liophilen Wasserhyazinthen-Extrakt in einem öligen Träger einschließt, der mindestens ein natürliches, raffiniertes oder nicht raffiniertes Pflanzenöl, ausgewählt aus dem Sonnenblumenöl, dem Olivenöl, dem Traubenkernöl und dem Jojobaöl, umfasst.

12. Nicht therapeutisches kosmetisches Verfahren für eine topische hydratisierende Wirkung auf der Haut, das darin besteht, auf die betroffenen Zonen der Haut eine kosmetische Zusammensetzung aufzutragen, die eine wirksame Menge einer öligen Zusammensetzung enthält, die mindestens einen liophilen Wasserhyazinthen-Extrakt einschließt, wobei die Zusammensetzung Anspruch 11 entspricht.

## Claims

1. The use of a lipophilic extract of *Eichhornia crassipes* in a cosmetic composition for topical application with a skin moisturizing action.

2. The use according to claim 1, **characterized in that** said extract is obtained by extraction sheltered from air.

3. The use according to claim 1, **characterized in that** the extract comprises:
| | |
|---|---|
| quercetin | min. 50 mg/kg |
| chlorogenic acid | min. 150 mg/kg |
| oleuropein | min. 250 mg/kg. |

4. The use according to any of the preceding claims, **characterized in that** said extract appears as an oily composition containing said lipophilic extract in an oily support comprising at least one natural vegetable oil, either refined or not, selected from sunflower oil, olive oil, grape pip oil and jojoba oil.

5. The use according to claim 4, characterizing that the vegetable oil is jojoba oil.

6. The use according to any of the preceding claims, **characterized in that** the extracts are obtained from the entire plant.

7. The use according to any of the preceding claims, **characterized in that** the cosmetic composition comprises between 0.05 and 15% by weight of lipophilic extract of *Eichhornia crassipes* based on the total weight of the cosmetic composition.

8. The use according to claim 7, **characterized in that** the cosmetic composition comprises from 0.2 to 10.0% of lipophilic extract of *Eichhornia crassipes* based on total weight of the cosmetic composition.

9. The use according to any of claims 1 to 6, **characterized in that** the cosmetic composition further comprises one or several secondary actives selected from mimosa seed extract, calendula seed extract, poppy (*Papaver rhoeas*) petal extract, apple tree leaf extract, cocoa cell extract, monomethylsilanol, proline, and shea butter.

10. The use according to any of the preceding claims, **characterized in that** the cosmetic composition is formulated as a lotion, a gel, an emulsion, a biphasic oil-in-water or water-in-oil emulsion, a body oil, a mask, an ointment, a pomade, a protective stick for lips, nanocapsules, liposomes or transdermal patch for topical application.

11. An oily composition **characterized in that** it contains at least one lipophilic extract of *Eichhornia crassipes* in an oily support comprising at least one natural vegetable oil, either refined or not, selected from sunflower oil, olive oil, grape pip oil and jojoba oil.

12. A non-therapeutic cosmetic method for providing a skin-moisturizing topical action, consisting of applying on the relevant skin areas a cosmetic composition containing an effective amount of an oily composition containing at least one lipophilic extract of *Eichhornia crassipes,* said composition being as defined in claim 11.
